(19) **Europäisches Patentamt / European Patent Office / Office européen des brevets**

(11) **EP 1 860 137 A1**

(12) **EUROPEAN PATENT APPLICATION**
published in accordance with Art. 158(3) EPC

(43) Date of publication:
**28.11.2007 Bulletin 2007/48**

(21) Application number: **06713969.1**

(22) Date of filing: **17.02.2006**

(51) Int Cl.:
***C08J 3/12*** *(2006.01)*          ***A61K 8/89*** *(2006.01)*
***A61Q 1/00*** *(2006.01)*

(86) International application number:
**PCT/JP2006/302828**

(87) International publication number:
**WO 2006/088130 (24.08.2006 Gazette 2006/34)**

(84) Designated Contracting States:
**DE ES FR GB IT**

(30) Priority: **17.02.2005 JP 2005040463**

(71) Applicant: **Shiseido Company, Limited**
**Chuo-ku**
**Tokyo 104-8010 (JP)**

(72) Inventors:
• **OGAWA, Katsuki,**
  **SHISEIDO RESEARCH CENTER**
  **Yokohama-shi, Kanagawa, 224-8558 (JP)**
• **YAGI, Katsuhiko,**
  **SHISEIDO RESEARCH CENTER**
  **Yokohama-shi, Kanagawa, 224-8558 (JP)**
• **OHNO, Mamoru**
  **Shiga, 5200842 (JP)**

(74) Representative: **Henkel, Feiler & Hänzel**
**Patentanwälte**
**Maximiliansplatz 21**
**80333 München (DE)**

(54) **SILICONE ELASTOMER COMPOSITE POWDER, METHOD FOR PRODUCING SILICONE ELASTOMER COMPOSITE POWDER AND COSMETIC**

(57)    The present invention provides a silicone elastomer composite powder with excellent flowability and dispersibility. Also, the present invention provides a method for producing a silicone elastomer composite powder, comprising a step of converting a mixture of a silicone elastomer powder and a clay mineral to the composite powder with a high-speed rotation dispersing machine under a condition of a peripheral speed of 40 m/sec or higher and a Froude number of 70 or higher, wherein a surface of the silicone elastomer powder is coated with the clay mineral, a specific volume of the composite powder is 2.50 ml/g or less, and an oil absorption amount of the composite powder is 180 g/100 g or less. Furthermore, the present invention provides a cosmetic without sticky feeling inherent in the silicone elastomer powder, and excellent in the feeling during use and in impact resistance.

**EP 1 860 137 A1**

**Description**

**RELATED APPLICATIONS**

This application claims the priority of Japanese Patent Application No. 2005-40463 filed on February 17, 2005, which is incorporated herein by reference.

**FIELD OF THE INVENTION**

The present invention relates to a new silicone elastomer composite powder, and in particular, relates to a silicone elastomer composite powder with excellent flowability and dispersibility, to the method for producing the same, and to cosmetics comprising the silicone elastomer composite powder.

**BACKGROUND ART**

Silicone elastomer powder is a spherical powder in which primary particles are in the range of 2-15 $\mu$m. Because the silicone elastomer powder has a dimethylsiloxane backbone in the molecule, it has excellent properties in that no sticky feeling is generated and it can impart lubricity and spreadability to cosmetics (refer to patent literatures 1 and 2, for example).

However, this silicone elastomer powder has a highly botryoidally-aggregated structure, and it has poor flowability and poor dispersibility Thus, when silicone elastomer powder is blended in cosmetics, sufficient dispersion is difficult to achieve. As a result, there is a problem that aggregates are generated in cosmetics as if they were foreign matters, and the cosmetics were poor in the extensibility and smoothness in feeling of use. In addition, when the dispersibility of silicone elastomer powder is poor, the internal strain is generated. As a result, when it is used in a compact powder foundation, the impact resistance could be extremely poor, leading to the poor portability of product.

Thus, various methods are proposed to reduce the aggregation of silicone elastomer powder.
Examples of a method using stirring and mixing actions includes stirring and mixing methods with the use of a mortar, mincing machine, ball mill, Henschel mixer, Nauta mixer, Redige mixer, a V-type mixer, etc. As a method using impact action, impacting methods with a hammer mill or a pin mill are also proposed (refer to patent literatures 3-5, for example).
Among the formers, however, especially in the stirring and mixing methods with the use of a mortar, mincing machine, or a ball mill, the shearing force is small. Thus, static electricity is generated during long-time mixing to problematically cause reaggregation. The latter impacting methods with a hammer mill or a pin mill have issues in that the rubber elasticity of silicone elastomer powder absorbs an impact force, whereby the pulverization is insufficient.

As a method to remedy these shortcomings, the method in that silicone elastomer powder is confined in a closed space and sheared to disperse the aggregates with the shearing stress. This method can be realized with the use of a millstone; however, the millstone method is unsuitable as an industrial method. To apply such a method in industrial production, the use of a closed multi-stage shear extruder is proposed in patent literatures 6 and 7.
The productivity can be improved by the method with a closed multi-stage shear extruder than the millstone method; however, troublesome handling is involved. As a result, there have been some issues in that the industrial-scale productivity was low and the production cost was high.

Therefore, as a method to increase productivity, the use of dry crushers, which have both a shearing mechanism and a sorting mechanism, such as a cutter mill, a turbo mill, or an impeller mill was proposed in patent literature 8.
This method involves two processes; the first process is pretreatment, in which silicone elastomer powder and an inorganic mineral are roughly mixed beforehand, and the second process is dispersion with the dry crusher. As a result, two kinds of apparatuses are necessary. When a cutter mill is used as a dry crusher, there is a cumbersome operation issue in that the silicone composite powder deposited inside the apparatus should be scraped down, and there is also a quality issue in that the deposit gets mixed in the product.

Patent literature 1: Japanese Unexamined Patent Publication No. S63-77942
Patent literature 2: Japanese Unexamined Patent Publication No. S64-70558
Patent literature 3: Japanese Patent No. 3273573
Patent literature 4: Japanese Unexamined Patent Publication No. H09-208709
Patent literature 5: Japanese Unexamined Patent Publication No. H10-36219
Patent literature 6: Japanese Unexamined Patent Publication No. H05-305223
Patent literature 7: Japanese Patent No. 3126553
Patent literature 8: Japanese Patent No. 3442698

## DISCLOSURE OF THE INVENTION

### PROBLEM TO BE SOLVED BY THE INVENTION

As described above, the conventional techniques have been largely empirical methods. Thus, an engineering approach to improve the aggregation of silicone elastomer powder, as an industrial method, has not been adequately investigated. That is to say, although the silicone elastomer powder was produced under the control of operation conditions such as the rotation number and the peripheral speed, when the type of an apparatus was changed or when, even for the same type of an apparatus, an experimental apparatus was scaled up to an industrial-scale production apparatus, it has been difficult to define suitable operation conditions, and the production of consistent-quality silicone elastomer powder has been difficult.

In addition, the obtained silicone elastomer powder has been evaluated by a qualitative method such as electron microscopy; thus, there is an issue in that the product quality control has not been satisfactory.

The present invention was made in view of the above-described circumstances. Thus, an object of the invention is to provide a silicone elastomer composite powder with excellent flowability and dispersibility by pulverizing a highly aggregated silicone elastomer powder. Another object is to provide a cosmetic exhibiting good feeling during use without sticky feeling inherent in the silicone elastomer powder, and making good use of the properties of the silicone elastomer powder such as lubricity and spreadability.

### MEANS TO SOLVE THE PROBLEM

The present inventors have diligently studied to provide a silicone elastomer composite powder with excellent flowability and dispersibility by pulverizing a highly aggregated silicone elastomer powder. As a result, the present inventors have found that it was possible to consistently produce a pulverized silicone elastomer composite powder with excellent flowability and dispersibility under a suitable production condition. In the production condition, the dimensionless Froude number, which is the index that controls the powder movement in a high-speed dispersion mixer, was taken into consideration in addition to the conventional production factors, namely, the rotation speed or the peripheral speed of rotating blades.

The present inventors have also found that a silicone elastomer composite powder with consistent quality could be provided by conducting quantitative evaluations such as the specific volume measurement and the oil absorption amount measurement instead of the conventional qualitative evaluation such as the evaluation with scanning electron microscopy

Thus, the present invention relates to a silicone elastomer composite powder, wherein a surface of a silicone elastomer powder is coated with a clay mineral, a specific volume of the composite powder is 2.50 ml/g or less, and an oil absorption amount of the composite powder is 180 g/100 g or less.

The method for producing the silicone elastomer composite powder comprises a step of converting a mixture of a silicone elastomer powder and a clay mineral to the composite powder, with a high-speed rotation dispersing machine under a condition of a peripheral speed of 40 m/sec or higher and a Froude number of 70 or higher.

In addition, the present invention provides a cosmetic comprising the above-described silicone elastomer composite powder.

### EFFECT OF THE INVENTION

The present invention can provide a silicone elastomer composite powder with excellent flowability and dispersibility by the pulverization of a highly aggregated silicone elastomer powder. In addition, the cosmetics of the present invention cause no sticky feeling inherent in the silicone elastomer powder, and make good use of the properties of the silicone elastomer powder such as lubricity and spreadability. Thus, the cosmetics are excellent in feeling during use and in impact resistance.

### BRIEF DESCRIPTION OF THE DRAWINGS

Fig. 1 shows a scanning electron microphotography of the silicone elastomer composite powder (which obtained in Production Example 1 described later).

Fig. 2 shows a scanning electron microphotography of the silicone elastomer composite powder (which obtained in Comparative Production Example 3 described later).

Fig. 3 shows a scanning electron microphotography of the silicone elastomer composite powder (which obtained in Comparative Production Example 1 described later).

**BEST MODE FOR CARRYING OUT THE INVENTION**

In the following, the best mode for carrying out the present invention is described.

(SILICONE ELASTOMER POWDER)

The silicone elastomer powder used in the present invention is a silicone rubber synthesized by addition reaction curing, condensation reaction curing, radical reaction curing with an organic peroxide, or UV-radiation curing. In particular, a desirable silicone elastomer powder is formed by curing a silicone composition obtained by addition reaction curing or condensation reaction curing. The silicone elastomer may contain or may not contain a non-crosslinked oil. The details for these silicone elastomer powders are described in Japanese Patent No. 3535389.

As commercial products, there are the Torayfil E series from Dow Corning Toray Co., Ltd.. Specific examples include Torayfil E-505C, Torayfil E-506S, Torayfil E-507, and Torayfil E-508.
The average primary particle size of the silicone elastomer is preferably 0.1-100 µm, more preferably 2-15 µm. The silicone hardness is preferably less than 90 JIS A hardness, more preferably 5-80 JIS A hardness.

(CLAY MINERAL)

Examples of clay minerals include talc, sericite, kaolin, mica, and titanated mica. The average primary particle size of the clay mineral is preferably 0.01-100 µm, more preferably 0.01-10 µm.

The weight ratio of the silicone elastomer powder and the clay mineral is preferably 98:2-10:90, more preferably 80:20-20:80. If there is too much silicone elastomer powder with respect to the clay mineral, huge aggregates of the silicone elastomer cannot be satisfactorily pulverized. If there is too little silicone elastomer powder, the effect of the powdery property of the clay mineral will be strong, whereby the lubricity and spreadability will be lost.

(SPECIFIC VOLUME)

The specific volume of the silicone elastomer composite powder of the present invention is 2.50 ml/g or less, preferably 1.0-2.3 ml/g. If the specific volume exceeds 2.50 ml/g, there is a problem in that aggregates remain due to the insufficiency of the pulverization of the silicone elastomer. If the specific volume is less than 1.0 ml/g, the structure of the silicone elastomer may be destroyed to spoil the rubber elasticity. The specific volume is used as one of the dispersibility indicators for the silicone elastomer composite powder.

For the measurement of the specific volume, an A.B.D. Powder Tester from Tsutsui Rikagaku Kikai Co., Ltd. was used. A composite powder is filled to the top of a cell assembly (inner dimensions: Φ474×115, separable to two stages), and the cell assembly is vibrated for 3 minutes. The top cell is removed, and the composite powder remaining in the bottom cell is scraped with a special knife so that the top will be even level with the top surface of the cell. The weight of the remaining composite powder in the bottom cell is measured to obtain the specific volume. The following equation (1) is used for calculation.

$$\text{Specific volume (ML/g)} = \text{volume of bottom cell (100 ML)} / (\text{weight of remaining composite powder with bottom cell (g)} - \text{weight of cell (g)}) \quad \text{----- (1)}$$

(OIL ABSORPTION AMOUNT)

The oil absorption amount of the silicone elastomer composite powder of the present invention should be 180 g/100 g or less, preferably 130-170 g/100 g. If the oil absorption amount is more than 184 g/100 g, the pulverization of silicone elastomer is not satisfactory, and the aggregates have voids that can absorb oil. Thus, there is a problem in that excess oil is necessary when the powder is blended into cosmetics. The oil absorption amount is used as one of the dispersibility indicators, as well as the specific volume, for the silicone elastomer composite powder.

The measurement method of the oil absorption amount is described in JIS K-5101. In the method, 1 g of the silicone elastomer composite powder is accurately weighed and placed on a glass plate. Dimethylsilicone oil (SH200C-5CS from Dow Corning Toray Co., Ltd.) is dropped, little by little, at the center of the composite powder. They are kneaded together after each addition of the oil with a spatula. The end point is set at the point immediately before fluidization takes place after the entirety becomes pasty. The following equation (2) is used for calculation.
$$\text{Oil absorption amount (g/100g)} =$$

$$(\text{SH200C-5CS (g)} / \text{weight of sample (g)} ) \times 100 \quad \text{----- (2)}$$

(PRODUCTION METHOD OF THE SILICONE ELASTOMER COMPOSITE POWDER)

In the present invention, a highly aggregated silicone elastomer powder is pulverized to obtain a silicone elastomer composite powder with excellent flowability and dispersibility. For that, the production process is controlled under a suitable condition, in which the dimensionless Froude number, which is the index that controls the powder movement in a high-speed dispersion mixer, is taken into consideration in addition to the conventional factors, namely, the rotation speed or the peripheral speed of rotating blades. Also, the product quality is controlled by quantitative evaluations such as a specific volume measurement and an oil absorption amount measurement in addition to the conventional qualitative evaluation by scanning electron microscopy. Thus, a silicone elastomer composite powder with consistent quality is produced.
More specifically, a mixture of a silicone elastomer powder and a clay mineral is converted into a composite powder with a high-speed rotation dispersing machine under the condition of a peripheral speed of 40 m/sec or higher and a Froude number of 70 or higher. The blending ratio of a silicone elastomer powder and a clay mineral is preferably 98: 2-10:94, more preferably 80:20-20:80.
A high-speed rotation dispersing machine used in the present invention is a dry-type high-speed dispersion mixer with a performance of a peripheral speed of 40 m/sec or higher and a Froude number of 70 or higher. The apparatus can provide a sufficient shearing force and an impact force to the clay mineral and the silicone elastomer powder and pulverize the highly aggregated silicone elastomer. Thus, the apparatus can produce a composite powder in which the silicone elastomer is coated with the clay mineral.
In the structure of a high-speed dispersion mixer used in the present invention, there are stirring blades vertically positioned at the center of the bottom section of the dispersion mixer tank. The dispersion mixer tank can be vertically cylindrical, horizontally cylindrical, spherical, etc.. Although any shape can be employed for the formation of composite powder, a vertically cylindrical or spherical shape is desirable to take out the product.
In the following section, an example of the detailed structure for a dry-type high-speed dispersion mixer used in the present invention is described.
The bottom of the tank is horizontal and circular, and a revolving shaft is vertically placed at the center of the tank bottom so that the powders to be treated can smoothly flow without staying. Here the powders to be treated mean a silicone elastomer powder, clay mineral particles, and a mixture thereof or a composite powder thereof.
In order to pulverize the aggregates of the powder, rotating blades are installed at the bottom section of the shaft, and auxiliary rotating blades are installed at the top section of the shaft. Thus, a shearing force and an impact force are generated with the high-speed rotation of the shaft.
In order to remove the powder-generated frictional heat due to the high-speed rotation, an external jacket is installed to the treatment apparatus, and the cooling is achieved with water or a cooling medium. In addition, the release of the frictional heat is promoted by passing gas from the tank bottom along the powder inner wall.
An exhaust pipe is installed, from the tank top, on the conical revolving shaft, and only the gas separated from the powders enters into the exhaust pipe. Then, the gas is released to the outside through the filter that is installed at the very top of the exhaust pipe.
The production method of a silicone elastomer composite powder of the present invention is further detailed. The process of the composite powder formation preferably comprises a process in which the low-speed rotation is performed at a peripheral speed, at the tips of the rotating blades, of 20 m/sec or less, and a Froude number of 30 or less, and a process in which the high-speed rotation is performed at a peripheral speed, at the tips of the rotating blades, of 40 m/sec or higher and a Froude number of 70 or higher.
In the first process of the low-speed rotation, the powders to be treated are loaded into the apparatus, and the revolving shaft is rotated for a few minutes to mix the clay mineral and the silicone elastomer powder and to generate static electricity on the powders. The clay mineral particles enter into the voids of the aggregated silicone elastomer powder, which has enlarged to a few hundred $\mu$m size, sometimes to a few mm size. Then enlarged aggregates change to smaller aggregates with a size of ca. 100 $\mu$m. The surface of the 100 $\mu$m aggregates are softly coated with the clay mineral particles, and a light dry fine powder, in appearance, is obtained.
The above treatment condition is at a low-speed rotation, and the peripheral speed at the tips of the rotating blades is 20 m/sec or less and the Froude number is 30 or less.

Subsequently, the second process, in which the blades are rotated at a high speed, is set in operation. In this process, the powders to be treated are elevated along the surface of the cylindrical wall by the centrifugal force of high-speed rotating blades and by the gas flow. Then the powders collide against the section near the top of the tank or collide with the exhaust pipe located at the top of the tank. When the inertial force, which the powders have received during elevation, becomes smaller than the gravitational force, the elevation speed is lost and the powders fall to the bottom.

When the powders fall to the bottom of the tank and to the rotating blades, the powders again receive energy from the centrifugal force of the rotating blades and the gas flow. As a result, the above-described powder circulation is repeated. The installed exhaust pipe has a function to separate the powders from the flowing gas. The powders and the gas that have descended along the periphery of the exhaust pipe reach the bottom of the exhaust pipe. Only the gas enters the exhaust pipe, and it is released to the outside through a filter located at the top of the exhaust pipe.

There are no angular sections inside the apparatus, and the structure is nearly spherical so that the powders can smoothly flow through the space. During the operation, the powders constantly flow without staying on the inner wall surface of the apparatus. Even when the powders happen to be deposited inside the apparatus, the deposit is removed by the self-cleaning action of the continuously flowing powders. Thus, the shearing force and the impact force are repeatedly provided to the powders by high-speed rotation force. The powders with the size of ca. 100 $\mu$m are pulverized, and the pulverization is accelerated with the passage of time. Thus, the silicone elastomer composite powder with the size of 2-20 $\mu$m, which is close to the primary particle size, is formed.

In this process condition, the peripheral speed at the tips of the rotating blades is preferably 40 m/sec or higher and the Froude number is preferably 70 or higher; more preferably, the peripheral speed is 50 m/sec or higher and the Froude number is 80 or higher.

Generally, the Froude number is large for a small apparatus; however, the peripheral speed is small. On the contrary, the Froude number is small for a large apparatus; however, the peripheral speed is large.

In order to pulverize the silicone elastomer powder and obtain a silicone elastomer composite powder coated with a clay mineral, it is necessary to select an apparatus that can satisfy the peripheral speed at the tips of the rotating blades and also the Froude number. In particular, it is important to consider these characteristics when the apparatus is scale-up. In reality, the selection of a dry-type high-speed dispersion mixer with a performance of a peripheral speed of 40 m/sec or higher and a Froude number of 70 or higher has a limitation. In the commercially-available Henschel mixer for mass production, it is possible to achieve a peripheral speed of 40 m/sec; however, it is difficult to achieve a Froude number of 70 or higher.

The Froude number is explained as follows in Terminology Dictionary of Powder Technology (Funtai Kogaku Yogo Jiten) (second edition; edited by the Society of Powder Technology, Japan; published by the Nikkan Kogyo Shimbun, Ltd.).

The Froude number (Froude Number $Fr = N^2R/g$) is a dimensionless number that represents the effect of the gravitational force during the movement of fluid or particles. The Froude number is defined as the ratio of the inertial force with respect to the gravitational force that operate on the fluid etc. (centrifugal force/gravitational force). Here, N represents the number of rotations, R represents the turning radius, and g represents the gravitational acceleration. If the Froude numbers are identical, the movements are dynamically similar. When the dynamic similarity is considered for the pressure, force, impact force, etc., this dimensionless Froude number is used. For practical purposes, it is applied in the scale-up of powder handling.

In the present invention, the composite powder formation is conducted using a high-speed rotation dispersing machine under a condition of the Froude number of 70 or higher, preferably 80 or higher and 300 or lower. If the Froude number is too small, the compressive force and impact force are not sufficient; as a result, it is insufficient for a clay mineral to enter the aggregates of the silicone elastomer and to pulverize them. The peripheral speed should be 40 m/sec or higher, preferably 50 m/sec or higher and 120 m/sec or lower. If the peripheral speed is too small, the shearing force is not sufficient and it is difficult to disperse the aggregates of the silicone elastomer with the shearing force.

The dispersibility of the obtained silicone elastomer composite powder can be quantitatively evaluated by a specific volume.

Highly aggregated silicone elastomer powder is bulky, and it has a large specific volume. However, the specific volume decreases by forming a composite powder with clay mineral particles. The trend of decrease is complicated. The decreasing curve shows a maximum during the process of the composite powder formation. Then the specific volume monotonously decreases, and from a certain value, shows a trend of a mild decrease. The results of the specific volume behavior together with the observation with a scanning electron microscope are as follows.

In the mixing process, clay mineral particles enter into the voids of the aggregated silicone elastomer powder, which has enlarged to a few hundred $\mu$m size, sometimes to a few mm size. Then the enlarged aggregates change to smaller aggregates with a size of ca. 100 $\mu$m. The surface of the 100 $\mu$m aggregates are softly coated with the clay mineral particles, and a light dry fine powder, in appearance, is obtained. However, the size of the silicone composite powder is still ca. 100 $\mu$m, and the specific volume is 1.9-2.0 ml/g at this stage.

In the subsequent composite powder formation process, the silicone composite powder with the size of ca. 100 $\mu$m is pulverized. Thus dispersed silicone composite powder with the size of 2-20 $\mu$m is coated with the clay mineral; however, some aggregates are still left. At this state, the specific volume increases and the maximum value reaches 3.0-3.5 ml/g. By further advancing the composite powder formation, a silicone elastomer composite powder, in which the powder is coated with clay mineral particles that are pulverized close to the primary particle size, is formed. At this stage, the specific volume monotonously decreases, and a slow decrease takes place from a certain constant value (2.0-1.6 ml/g).

The dispersibility of the obtained silicone elastomer composite powder can be quantitatively evaluated with the oil absorption amount in addition to the above-described specific volume.

As described above, the decreasing curve of the specific volume shows a maximum during the process of the composite powder formation; thus, the specific volume does not decrease monotonously. Therefore, the composite powder formation cannot be controlled sufficiently by the specific volume only, and the oil absorption amount is used at the same time.

The oil absorption amount, unlike the specific volume, monotonously decreases during the pulverization of the silicone elastomer powder and with the progress of composite powder formation with the clay mineral particles. It is considered that the amount of absorbable oil into the silicone elastomer decreases by the pulverization.

The qualitative analysis of the composite powder formation for the obtained silicone elastomer composite powders can be performed by the conventional observation with a scanning electron microscope.

(COSMETICS)

According to the present invention, the cosmetics in which the above-described silicone elastomer composite powder is blended can be provided.

The amount of the above-described composite powder that is blended in the cosmetics of the present invention is not limited in particular; however, it is normally 0.5-50 weight %.

The cosmetics of the present invention include W/O emulsion cosmetics, oil-based cosmetics, solid-type powder cosmetics, etc., and preferably they are makeup cosmetics.

In addition, the commonly used components in constituting these types of cosmetic can be blended. Examples thereof include higher alcohols; lanolin derivatives; protein derivatives; oil components such as polyethylene glycol fatty acid esters, silicone oils, paraffin oils, and fluorinated oils; moisturizing components such as propylene glycol, glycerin, and polyethylene glycol; oil-soluble polymers; water-soluble polymers; ion-exchanged water; alcohols; extender pigments such as talc, mica, and kaolin; iron oxide; spherical powder; antiseptics; disinfectants; pH adjusting agents; antioxidants; coloring matters; and perfumes.

**EXAMPLES**

The present invention will hereinafter be described in further detail by examples. However, the present invention is not limited by these examples. The blending amount was expressed in the weight part or weight % unless otherwise noted.

EXAMPLE A (PRODUCTION OF SILICONE ELASTOMER COMPOSITE POWDER)

Example (A-1) (Production Examples 1-5 and Comparative Production Examples 1-6)

As the silicone elastomer powder, 40 parts of Torayfil E-506S from Dow Coming Toray Co., Ltd. was used. As the clay mineral, 60 parts of talc (Asada Milling Co., Ltd. JA-68 R, average particle size: 9.0-12.0 $\mu$m) was used.

These raw materials were put into the apparatus described in Table 1. Cooling water was passed through the jacket section of the apparatus, and the apparatus was rotated at a low peripheral speed of 20 m/sec for 3 minutes in order to mix the materials to be treated.

Subsequently, the apparatus was rotated at a higher speed, under various conditions described in Table 1, for 60 minutes to treat the materials. When the high-speed rotation, in which the peripheral speed was 40 m/sec or higher and the Froude number was 70 or higher, was reached, the powder temperature rapidly increased. The temperature became constant, after 2-3 minutes, in the range of 40-60 ˚C. The high-speed rotation was terminated after 60 minutes, and the low-speed rotation was continued at a peripheral speed of 20 m/sec or less to cool the composite to room temperature. Then the composite was collected. There was almost no deposit left in the tank. The state of composite powder formation was observed with a scanning electron microscope, and the specific volume and the oil absorption amount were determined.

Example (A-2) (Production Examples 6-7 and Comparative Examples 7-8)

A silicone elastomer powder having 14 JIS A rubber hardness was produced according to the method described in the "Production Method of Organopolysiloxane Elastomer Spherical Powder" of Japanese Patent No. 3535359. This silicone elastomer powder in the amount of 35 parts was used. As the clay mineral, 65 parts of tale (Asada Milling Co., Ltd., JA-13R, average particle size: 5.0-8.0 $\mu$m) was used.

These raw materials were put into an apparatus described in Table 2. Cooling water was passed through the jacket section of the apparatus, and the apparatus was rotated at a low peripheral speed of 20 m/sec for 3 minutes in order to mix the materials to be treated.

Subsequently, the apparatus was rotated at a high speed under various conditions described in Table 2, to treat the materials for 60 minutes. When the high-speed rotation, in which the peripheral speed was 40 m/sec or higher and the Froude number was 70 or higher, was reached, the powder temperature rapidly increased. The temperature became constant, after 2-3 minutes, in the range of 40-60 ˚C. The high-speed rotation was terminated after 90 minutes, and the low-speed rotation was continued at a peripheral speed of 20 m/sec or less, to cool the composite to room temperature.

Then the composite was collected. There was almost no deposit left in the tank. The state of composite powder formation was observed with a scanning electron microscope, and the specific volume and the oil absorption amount were determined.

Example (A-3) (Production Examples 8 and 9 and Comparative Examples 9 and 10)

A silicone elastomer powder having 6 JIS A rubber hardness was produced according to the method described in the "Production Method of Organopolysiloxane Elastomer Spherical Powder" of Japanese Patent No. 3535389. This silicone elastomer powder in the amount of 35 parts was used. As the clay mineral, 65 parts of talc (Asada Milling Co., Ltd., JA-13R, average particle diameter: 5.0-8.0 $\mu$m) was used.

These raw materials were put into an apparatus described in Table 3. Cooling water was passed through the jacket section of the apparatus, and the apparatus was rotated at a low peripheral speed of 20 m/sec for 3 minutes in order to mix the materials to be treated.

Subsequently, the apparatus was rotated at a high speed under various conditions described in Table 3, to treat the materials for 60 minutes. When the high-speed rotation, in which the peripheral speed was 40 m/sec or higher and the Froude number was 70 or higher, was reached, the powder temperature rapidly increased. The temperature became constant, after 2-3 minutes, in the range of 40-60 ˚C. The high-speed rotation was terminated after 120 minutes, and the low-speed rotation was continued at a peripheral speed of 20 m/sec or less to cool the composite to room temperature. Then the composite was collected. There was almost no deposit in the tank. The state of composite powder formation was observed with a scanning electron microscope, and the specific volume and the oil absorption amount were determined.

The state of the composite powder by the observation with a scanning electron microscope was evaluated according to the three-grade evaluation described below, and the results are shown in Tables 1-3.

○: Dispersed silicone elastomer powder with the size of 2-20 $\mu$m is coated with talc.

Δ: Dispersed silicone elastomer powder with the size of 2-20 $\mu$m is coated with talc; however, some aggregates are still left.

✕: Highly aggregated silicone elastomer powder is coated with talc.

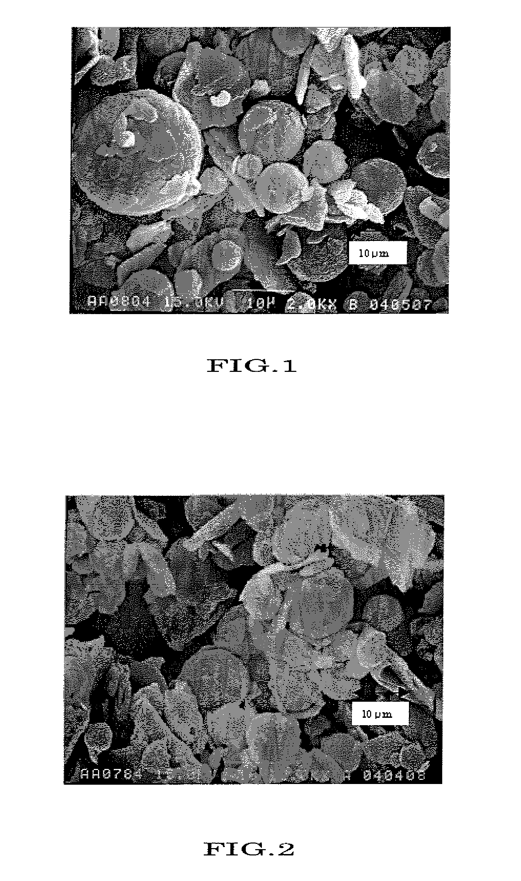

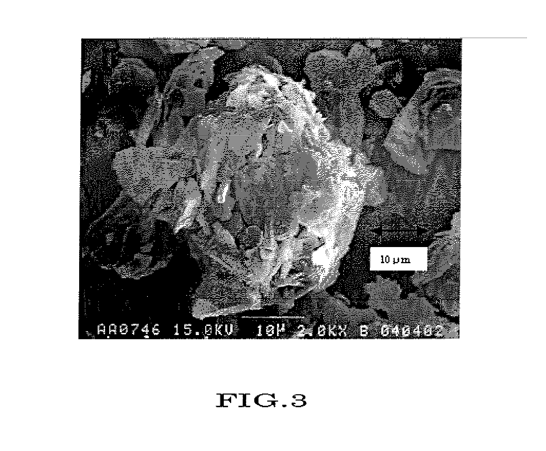

A scanning electron microphotography of Production Example 1, in which the state of the composite powder corresponds to ○, is shown in Fig. 1. A scanning electron microphotography of Comparative Production Example 3, in which the state of the composite powder corresponds to Δ, is shown in Fig. 2. A scanning electron microphotography of Comparative Production Example 1, in which the state of the composite powder corresponds to ✕, is shown in Fig. 3.

## TABLE 1

| Example (A-1) | | Type of apparatus | Peripheral speed | Froude No. Fr | Specific volume | Oil absorption amount | Results of SEM observation for composite state |
|---|---|---|---|---|---|---|---|
| | | | m/sec | $(n/60)^2 r/g$ | ml/g | g/100g | |
| Production Ex. | Production Ex. 1 | High-speed rotation dispersing machine(Type A) | 76 | 240 | 2.20 | 143 | ○ |
| | Production Ex. 2 | High-speed rotation dispersing machine(Type A) | 63 | 170 | 2.40 | 145 | ○ |
| | Production Ex. 3 | High-speed rotation dispersing machine(Type B) | 100 | 184 | 1.97 | 140 | ○ |
| | Production Ex. 4 | High-speed rotation dispersing machine(Type B) | 87 | 142 | 2.20 | 145 | ○ |
| | Production Ex. 5 | High-speed rotation dispersing machine(Type C) | 105 | 102 | 1.95 | 140 | ○ |
| Comparative Ex. | Comp. Production Ex. 1 | High-speed rotation dispersing machine(Type A) | 25 | 27 | 2.70 | 198 | × |
| | Comp. Production Ex. 2 | High-speed rotation dispersing machine(Type B) | 58 | 63 | 3.36 | 182 | × |
| | Comp. Production Ex. 3 | High-speed rotation dispersing machine(Type C) | 73 | 50 | 2.80 | 162 | △ |
| | Comp. Production Ex. 4 | High-speed rotation dispersing machine(Type C) | 50 | 23 | 3.02 | 184 | × |

| Comp. Production Ex. 5 | Speed mixer | 27 | 7 | 2.30 | 230 | × |
| Comp. Production Ex. 6 | Henschel mixer | 35 | 10 | 1.90 | 240 | × |

## TABLE 2

| Example (A-2) | | Type of apparatus | Peripheral speed | Froude No. $F_r$ | Specific volume | Oil absorption amount | Results of SEM observation for composite state |
|---|---|---|---|---|---|---|---|
| | | | m/sec | $(n/60)^2 r/g$ | ml/g | g/100g | |
| Production Ex. | Production Ex. 6 | High-speed rotation dispersing machine(Type B) | 94 | 163 | 1.66 | 162 | ○ |
| | Production Ex. 7 | High-speed rotation dispersing machine(Type C) | 103 | 97 | 1.66 | 151 | ○ |
| Comp. Ex. | Comp. Production Ex. 7 | High-speed rotation dispersing machine(Type B) | 58 | 63 | 2.20 | 210 | × |
| | Comp. Production Ex. 8 | High-speed rotation dispersing machine(Type C) | 50 | 23 | 1.99 | 203 | × |

## TABLE 3

| Example (A-2) | | Type of apparatus | Peripheral speed | Froude No. Fr | Specific volume | Oil absorption amount | Results of SEM obsarvation for composite state |
|---|---|---|---|---|---|---|---|
| | | | m/sec | $(n/60)^2 r/g$ | ml/g | g/100g | |
| Production Ex. | Production Ex. 8 | High-speed rotation dispersing machine(Type B) | 94 | 163 | 2.05 | 168 | ○ |
| | Production Ex. 9 | High-speed rotation dispersing machine(Type C) | 103 | 97 | 1.92 | 165 | ○ |

| | | | | | | | |
|---|---|---|---|---|---|---|---|
| Comp. Ex. | Comp. Production Ex. 9 | High-speed rotation dispersing machine(Type B) | 58 | 63 | 2.26 | 214 | × |
| | Comp. Production Ex. 10 | High-speed rotation dispersing machine(Type C) | 50 | 23 | 2.15 | 194 | × |

EXAMPLE B (PRODUCTION OF COSMETICS)

The cosmetics were prepared according to the formulations described below using the talc-coated silicone elastomer composite powder obtained in the above-described Example A

(Production Examples and Comparative Production Examples).

The evaluations of practical performance, hardness, and impact resistance for the present examples are as follows.
(1) Evaluation of practical performance
A sample was applied on the skin of 20 female panelists, and smoothness, softness, and rough texture were evaluated based on the following criteria.

(Evaluation criteria)

◎ : 17 or more panelists answered "good".
○ : 12-16 panelists answered "good".
Δ : 9-11 panelists answered "good".
× : 5-8 panelists answered "good".
× × : 4 or fewer panelists answered "good".

(2) Hardness

The hardness was measured by the following method.
The hardness was based on the values measured by a rheometer at 25 ˚C. (Measurement conditions: diameter of pressure sensitive axis: 2 mm, penetration speed: 2 cm/min, penetration depth: 1 mm, loaded weight: 2 kg, and the rheometer is from Fudou Manufacturing Co., Ltd.)

(3) Evaluation of impact resistance

A sample was pressed in a resin, and it was set in a compact container for cosmetics use to obtain a test sample. The test sample was horizontally dropped on an iron plate with a thickness of 20 mm from a height of 30 cm, and the number of drops till breakage was assessed as the impact resistance.

Example (B-1) (Examples 1-6 and Comparative Examples 1-7)

Example 1 and Comparative Examples 1-6

A powder foundation containing the talc-coated silicone elastomer composite powder that was obtained in Production Example 1 was produced, and it is shown in Example 1 of Table 4. Powder components and oil components of the formulation described below were mixed with a Henschel mixer, pulverized twice with a pulverizer, and dry-pressed in a resin inner dish.
On the other hand, in Comparative Examples 1-6, a powder foundation containing the talc-coated silicone elastomer composite powder that was obtained in Comparative Production Examples 1-6 (according to the method described in Japanese Patent No. 3442698) was produced.
The practical performance (smoothness, softness, and rough texture), hardness, and impact resistance for the obtained powder foundations were evaluated by the above-described methods. The results are shown together in Table 4.
TABLE 4

| Example (B-1) | Ex. 1 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Sericite | 7 | 7 | 7 | 7 | 7 | 7 | 7 |
| Synthetic mica | 10 | 10 | 10 | 10 | 10 | 10 | 10 |
| Talc | Balance | Balance | Balance | Balance | Balance | Balance | Balance |
| Titanium oxide | 12 | 12 | 12 | 12 | 12 | 12 | 12 |
| Red iron oxide | 0.8 | 0.8 | 0.8 | 0.8 | 0.8 | 0.8 | 0.8 |
| Yellow iron oxide | 2 | 2 | 2 | 2 | 2 | 2 | 2 |
| Black iron oxide | 0.1 | 0.1 | 0.1 | 0.1 | 0.1 | 0.1 | 0.1 |
| Silicone elastomer composite particle | 15 | 15 | 15 | 15 | 15 | 15 | 15 |
| Spherical nylon powder | 4 | 4 | 4 | 4 | 4 | 4 | 4 |
| Dimethylpolysiloxane | 3 | 3 | 3 | 3 | 3 | 3 | 3 |
| Ester oil | 2 | 2 | 2 | 2 | 2 | 2 | 2 |
| Squalane | 5 | 5 | 5 | 5 | 5 | 5 | 5 |
| Vaseline | 2 | 2 | 2 | 2 | 2 | 2 | 2 |
| Sorbitan sesquiisostearate | 1 | 1 | 1 | 1 | 1 | 1 | 1 |
| Paraben | Q.S. | Q.S. | Q.S. | Q.S. | Q.S. | Q.S. | Q.S. |
| Antioxidant | Q.S. | Q.S. | Q.S. | Q.S. | Q.S. | Q.S. | Q.S. |

(continued)

| Example (B-1) | Ex. 1 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Perfume | Q.S. | Q.S. | Q.S. | Q.S. | Q.S. | Q.S. | Q.S. |
| Smoothness | ◎ | ○ | ○ | ○ | ○ | ○ | Δ |
| Softness | ◎ | ○ | ○ | ◎ | ○ | ○ | Δ |
| Rough texture (foreign matters incorporation) | ◎ | Δ | × | Δ | Δ | × | × |
| Hardness | 51 | 47 | 42 | 48 | 49 | 50 | 44 |
| Impact resistance | 10 | 4 | 4 | 5 | 5 | 3 | 3 |

Example 2 and Comparative Example 7

A powder foundation containing the talc-coated silicone elastomer composite powder that was obtained in Production Example 2 was produced, and it is shown in Example 2 of Table 5. Powder components and oil components of the formulation described below were mixed with a Henschel mixer, pulverized twice with a pulverizer, and dry-pressed in a resin inner dish. On the other hand, in Comparative Examples 7, a powder foundation containing the silicone elastomer without composite formation was produced.

The practical performance, hardness, and impact resistance for the obtained powder foundations were evaluated by the above-described methods. The results are shown together in Table 5.

TABLE 5

| Example (B-1) | Ex.2 | Comp. Ex. 7 |
|---|---|---|
| Silicone-treated sericite | 13 | 13 |
| Silicone-treated mica | 8 | 8 |
| Silicon e-treated talc | Balance | Balance |
| Silicone-treated titanium oxide | 10 | 10 |
| Aluminum stearate-treated fine particle titanium oxide | 6 | 6 |
| Silicone-treated red iron oxide | 1.2 | 1.2 |
| Silicone-treated yellow iron oxide | 2.5 | 2.5 |
| Silicone-treated black iron oxide | 0.9 | 0.9 |
| Talc-coated silicone elastomer composite particle | 20 | - |
| Silicone elastomer | - | 20 |
| Spherical urethane elastomer | 3 | 3 |
| Paraben | Q.S. | Q.S. |
| Dimethylpolysiloxane | 4 | 4 |
| Methylphenylpolysiloxane | 3 | 3 |
| Octyl methoxycinnamate | 3 | 3 |
| Polyether silicone | 2 | 2 |
| Antioxidant | Q.S. | Q.S. |
| Perfume | Q.S. | Q.S. |
| Smoothness | ◎ | Δ |
| Softness | ◎ | Δ |

(continued)

| Example (B-1) | Ex.2 | Comp. Ex. 7 |
|---|---|---|
| Rough texture (foreign matters incorporation) | ◎ | × |
| Hardness | 76 | 52 |
| Impact resistance | 6 | 2 |

Example 3 Face powder

As Example 3, a face powder containing the talc-coated silicone elastomer composite powder that was obtained in Production Example 3 was produced. Powder components and oil components of the formulation described below were mixed with a Henschel mixer, pulverized twice with a pulverizer, and dry-pressed in a resin inner dish.
| (Formulation) | |
|---|---|
| Talc | Balance |
| Mica | 25 wt % |
| Zinc oxide | 5 |
| Fine particle titanium oxide | 3 |
| Talc-coated silicone elastomer composite powder | 8 |
| Vaseline | 1 |
| Squalane | 3 |
| Ester oil | 1 |
| Paraben | Q.S. |
| Antioxidant | Q.S. |
| Perfume | Q.S. |

Example 4 Face powder

As Example 4, a face powder containing the talc-coated silicone elastomer composite powder that was obtained in Production Example 4 was produced. Powder components and oil components of the formulation described below were mixed with a Henschel mixer, pulverized twice with a pulverizer, and filled in a container in a powdery state.
| (Formulation) | |
|---|---|
| Talc | Balance |
| Synthetic mica | 30 wt % |
| Flaky zinc oxide | 6 |
| Red iron oxide | 0.2 |
| Yellow iron oxide | 0.7 |
| Fine particle titanium oxide | 1 |
| Talc-coated silicone elastomer composite powder | 10 |
| Dimethylpolysiloxane | 7 |
| Paraben | Q.S. |
| Antioxidant | Q.S. |
| Perfume | Q.S. |

Example 5 O/W emulsified cream foundation

As Example 5, an O/W emulsified cream foundation containing the talc-coated silicone elastomer composite powder that was obtained in Production Example 5 was produced. The water phase and the surfactant described in the formulation described below were prepared, and then powder components were dispersed therein with a homomixer. The dispersion, with the oil components at 80˚C gradually added thereto, was emulsified with a homomixer. The resulting emulsion was filled into a container.

(Formulation)

| | |
|---|---|
| Talc | 5 wt% |
| Sericite | 7 |
| Talc-coated silicone elastomer composite powder | 4 |
| Red iron oxide | 0.3 |
| Yellow iron oxide | 1.2 |
| Black iron oxide | 0.6 |
| Spherical polyethylene powder | 6 |
| Squalane | 10 |
| Olive oil | 10 |
| Stearic acid | 2 |
| Glyceryl monostearate | 2 |
| POE(40) sorbitan monostearate | 2 |
| Glycerin | 5 |
| Triethanolamine | 0.8 |
| pH adjusting agent | Q.S. |
| Antiseptics | Q. S. |
| Ion-exchanged water | Balance |

Example 6 O/W emulsified liquid foundation

As Example 6, an O/W emulsified cream foundation containing the talc-coated silicone elastomer composite powder that was obtained in Production Example 1 was produced. The powder components were dispersed into oil components and surfactants described in the formulation described below with a homomixer. The dispersion, with the prepared water components gradually added thereto, was emulsified with a homomixer. The resulting emulsion was filled into a container.

(Formulation)

| | |
|---|---|
| Silicone-treated mica | 3 wt% |
| Silicone-treated red iron oxide | 1 |
| Silicone-treated yellow iron oxide | 3 |
| Silicone-treated black iron oxide | 0.1 |
| Talc-coated silicone elastomer composite powder | 10 |
| Spherical PMMA powder | 2 |
| Cyclomethicone | Balance |
| Dimethylpolysiloxane | 4 |
| Squalane | 1 |
| Polyether-modified silicone | 3 |
| Sorbitan sesquiisostearate | 1 |
| Dispersing assistant | Q.S. |
| Dipropylene glycol | 2 |
| Ion-exchanged water | 20 |
| Paraben | Q.S. |
| Antioxidant | Q.S. |
| Perfume | Q.S. |

Example (B-2) (Example 7-8 and Comparative Example 8-9)

According to the formulation of Table 6, a powder foundation of Examples 7-8 using the talc-coated silicone elastomer composite powder that was obtained in Production Examples 6-7 was produced. Powder components and oil components of the formulation described below were mixed with a Henschel mixer, pulverized twice with a pulverizer, and dry-pressed in a resin inner dish.

On the other hand, in Comparative Examples 7-8, a powder foundation containing the talc-coated silicone elastomer composite powder that was obtained in Comparative Production Examples 7-8 (according to the method described in Japanese Patent No. 3442698) was produced.

The practical performance (smoothness, softness, and rough texture), hardness, and impact resistance for the obtained powder foundations were evaluated by the above-described methods. The results are shown together in Table 6.

TABLE 6

| Example (B-2) | Ex. 7 | Ex. 8 | Comp. Ex. 8 | Comp. Ex. 9 |
|---|---|---|---|---|
| Fluorinated silicone-treated sericite | 20 | 20 | 20 | 20 |
| Fluorinated silicone-treated mica | 10 | 10 | 10 | 10 |
| Fluorinated silicone-treated talc | Balance | Balance | Balance | Balance |
| Barium sulfate | 7 | 7 | 7 | 7 |
| Fluorinated silicone-treated titanium oxide | 13 | 13 | 13 | 13 |
| Fluorinated silicone-treated red iron oxide | 0.6 | 0.6 | 0.6 | 0.6 |
| Fluorinated silicone-treated yellow iron oxide | 2.1 | 2.1 | 2.1 | 2.1 |
| Fluorinated silicone-treated black iron oxide | 0.1 | 0.1 | 0.1 | 0.1 |
| Silicone-treated fine particle titanium oxide | 4 | 4 | 4 | 4 |
| Silicone elastomer composite particle | 10 | 10 | 10 | 10 |
| Spherical PMMA powder | 3 | 3 | 3 | 3 |
| Dimethylpolysiloxane | 2 | 2 | 2 | 2 |
| Dimethylphenylpolysiloxane | 1 | 1 | 1 | 1 |
| Squalane | 1 | 1 | 1 | 1 |
| Octyl methoxycinnamate | 3 | 3 | 3 | 3 |
| Vaseline | 1 | 1 | 1 | 1 |
| Sorbitan sesquiisostearate | 1 | 1 | 1 | 1 |
| Paraben | Q.S. | Q.S. | Q.S. | Q.S. |
| Antioxidant | Q.S. | Q.S. | Q.S. | Q.S. |
| Perfume | Q.S. | Q.S. | Q.S. | Q.S. |
| Smoothness | ◎ | ◎ | Δ | Δ |
| Softness | ◎ | ◎ | ◎ | ◎ |
| Rough texture (foreign matters incorporation) | ◎ | ◎ | Δ | Δ |
| Hardness | 57 | 55 | 72 | 69 |
| Impact resistance | 7 | 8 | 3 | 4 |

Example (B-3) (Example 9-10 and Comparative Example 11-12)

According to the formulation of Table 7, a powder foundation of Examples 9-10 using the talc-coated silicone elastomer composite powder that was obtained in Production Examples 8-9 was produced. Powder components and oil components of the formulation described below were mixed with a Henschel mixer, pulverized twice with a pulverizer, and dry-pressed in a resin inner dish.

On the other hand, in Comparative Examples 10-11, a powder foundation containing the talc-coated silicone elastomer composite powder that was obtained in Comparative Production Examples 9-10 (according to the method described in

EP 1 860 137 A1

Japanese Patent No. 3442698) was produced.
The practical performance (smoothness, softness, and rough texture), hardness, and impact resistance for the obtained powder foundations were evaluated by the above-described methods. The results are shown together in Table 7.
TABLE 7

| Example (B-3) | Ex. 9 | Ex.10 | Comp. Ex. 10 | Comp. Ex. 11 |
|---|---|---|---|---|
| Branched alkylsilicone-treated sericite | 15 | 15 | 15 | 15 |
| Branched alkylsilicone-treated mica | 10 | 10 | 10 | 10 |
| Branched alkylsilicone-treated talc | Balance | Balance | Balance | Balance |
| Pearly pigment with red interference color | 3 | 3 | 3 | 3 |
| Branched alkylsilicone-treated titanium oxide | 12 | 12 | 12 | 12 |
| Branched alkylsilicone-treated red iron oxide | 0.6 | 0.6 | 0.6 | 0.6 |
| Branched alkylsilicone-treated yellow iron oxide | 2.1 | 2.1 | 2.1 | 2.1 |
| Branched alkylsilicone-treated black iron oxide | 0.1 | 0.1 | 0.1 | 0.1 |
| Silicone-treated fine particle titanium oxide | 5 | 5 | 5 | 5 |
| Silicone elastomer composite particle | 12 | 12 | 12 | 12 |
| Spherical silicone powder | 3 | 3 | 3 | 3 |
| Dimethylpolysiloxane | 3 | 3 | 3 | 3 |
| Ester oil | 2 | 2 | 2 | 2 |
| Squalane | 1 | 1 | 1 | 1 |
| Octyl methoxycinnamate | 2 | 2 | 2 | 2 |
| Vaseline | 1 | 1 | 1 | 1 |
| Sorbitan sesquiisostearate | 1 | 1 | 1 | 1 |
| Paraben | Q.S. | Q.S. | Q.S. | Q.S. |
| Antioxidant | Q.S. | Q.S. | Q.S. | Q.S. |
| Perfume | Q.S. | Q.S. | Q.S. | Q.S. |
| Smoothness | ◎ | ◎ | Δ | Δ |
| Softness | ○ | ○ | ○ | ○ |
| Rough texture (foreign matters incorporation) | ◎ | ◎ | × | × |
| Hardness | 45 | 43 | 57 | 55 |
| Impact resistance | 12 | 13 | 2 | 3 |

Claims

1. A silicone elastomer composite powder, wherein a surface of a silicone elastomer powder is coated with a clay mineral, a specific volume of the composite powder is 2.50 ml/g or less, and an oil absorption amount of the composite powder is 180 g/100 g or less.

2. A method for producing a silicone elastomer composite powder, comprising a step of converting a mixture of a silicone elastomer powder and a clay mineral to the composite powder with a high-speed rotation dispersing machine under a condition of a peripheral speed of 40 m/sec or higher and a Froude number of 70 or higher; and wherein a surface of the silicone elastomer powder is coated with the clay mineral, a specific volume of the composite powder is 2.50 ml/g or less, and an oil absorption amount of the composite powder is 180 g/100 g or less.

17

3. A cosmetic, comprising the silicone elastomer composite powder according to claim 1.

FIG.1

FIG.2

FIG.3

<div style="text-align: center">**INTERNATIONAL SEARCH REPORT**</div>

| | International application No. |
|---|---|
| | PCT/JP2006/302828 |

A. CLASSIFICATION OF SUBJECT MATTER
*C08J3/12*(2006.01), *A61K8/89*(2006.01), *A61Q1/00*(2006.01)

According to International Patent Classification (IPC) or to both national classification and IPC

B. FIELDS SEARCHED

Minimum documentation searched (classification system followed by classification symbols)
A61K8/89-899, C08J3/12-16

Documentation searched other than minimum documentation to the extent that such documents are included in the fields searched
Jitsuyo Shinan Koho         1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2001-114623 A  (Kanebo, Ltd.),<br>24 April, 2001 (24.04.01),<br>Claim 2; Par. No. [0010]<br>(Family: none) | 1-3 |
| A | JP 7-149914 A  (Kao Corp.),<br>13 June, 1995 (13.06.95),<br>Claims 1, 4; Par. No. [0012]<br>(Family: none) | 1-3 |

☐ Further documents are listed in the continuation of Box C.       ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered   to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 02 March, 2006 (02.03.06) | 14 March, 2006 (14.03.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

<div style="text-align: center">21</div>

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005040463 A
- JP 63077942 A
- JP 6470558 A
- JP 3273573 B
- JP H09208709 A
- JP H1036219 A
- JP H05305223 A
- JP 3126553 B
- JP 3442698 B
- JP 3535389 B
- JP 3535359 B